# EUROPEAN PATENT APPLICATION

(11) **EP 1 447 408 A1**
(43) Date of publication of application: **18.08.2004**
(21) Application number: 04250640.2
(22) Date of filing: 06.02.2004
(51) Int. Cl.: C07K 1/04, C07K 7/00, C07K 14/00

(54) **Amino acid loaded trityl alcohol resins, method of production of amino acid loaded trityl alcohol resins and therapeutics produced therewith**

(30) Priority: 12.02.2003 US 447202
(71) Applicant: Rohm and Haas Company, Philadelphia PA 19106-2399 (US)
(72) Inventor: Bohling, James Charles, Lansdale,Pennsylvania 19446 (US); Maikner, John Joseph, Quakertown, Pennsylvania 18951 (US); Kinzey, Marlin Kenneth, Philadelphia, Pennsylvania 19147 (US); Ziarno, Witold Andrew, Horsham, Pennsylvania 19044 (US)
(74) Representative: Kent, Venetia Katherine

(57) **Abstract**

The present invention relates to a process for making a biologically active substance or therapeutic agent free of a chlorotrityl chloride linker-resin. The process includes reacting an activated amino acid or activated amino acid derivative with a substituted or unsubstituted trityl alcohol resin to obtain a resin-CT-AA product; and, reacting the resin-CT-AA product with other building blocks of the biologically active substance or therapeutic agent to obtain said biologically active substance or therapeutic. A product created by this process is also provided herein. The invention is particularly useful to create T-20 and T-1249 therapeutics.

## Description

### BACKGROUND OF THE INVENTION

Various techniques exist to use solid phase peptide synthesis to build peptides having biological activity and other therapuetics. Currently, the resin of choice for building peptides is chlorotrityl chloride linker on 1% DVB polystyrene. These resins are known as CTC resins and are moisture sensitive and very expensive. 2'CTC can be purchased from Argonaut Technologies, Inc. at a price of $13,500/Kg in the 100g scale. (Argonaut Resins and Reagents Catalog, 2002 pg. 170.) Yet, the advances in peptide therapy require larger, reasonably priced quantities of resins to synthesize commercial quantities of therapeutic peptides, biologically active substances, and cost effective alternates to CTC resins.

Notwithstanding the high cost of CTC resins and other drawbacks of this peptide building platform, the art has attempted to improve on the techniques for preparing CTC solid supports. See, The Advanced Chem Tech Handbook, William D. Bennett et al., 1998 at pg 341 which suggests using 2 eq. of pyridine to thionyl chloride (SOCl₂). Pyridine is both toxic and foul smelling. Orosz et al. report the use of an excess of trimethylsilylchloride and dimethylsulfoxide followed by treatment with AcCl. See, Orosz et al. Tetrahedron Letters 39 (1998) 3241-3242. The Orosz process is expensive. It also requires extensive washes to remove dimethylsulfoxide (DMSO) residue. Harre et al. discloses washing the resin with an excess of N, N-dimethylformamide (DMF) then dichloromethane (DCM)and then treating with SOCl₂. Harre also points out the problems associated with this particular reaction. See, Harre et al. Reactive and Functional Polymers 41 (1999) 111-114. Sanghvi et al( US 6239220) discloses, in Example 1, the conversion of dimethoxytritylalcohol to dimethoxytrityl chloride using acetyl chloride (AcCl).

Example 2 of US Patent No. 5,563,220 describes the overnight use of 2-chlorobenzoylchloride to form the keto resin, which is subsequently converted to the alcohol resin. The alcohol resin is converted to the chloride form by treatment with acetyl chloride. These process requires a CTC resin prior to the loading of an amino acid thereon. There exists a need in the art for a process that eliminates the use of CTC resins in the creation of biologically active substances and peptide therapeutics.

Applicants have discovered a method for producing solid supported loaded amino acid resins that overcome the problems in the art and that meets the demand for a robust process to efficiently produce large scale quantities. Further, the method of the present invention results in substantially decreased cycle times, decreased raw material usage and avoids handling issues associated with moisture sensitive solids when compared to methods using CTC loaded resins, and, in one variant, totally eliminates the use of CTC resins.

Prior to the present invention, the initial resin was loaded with a first amino acid by contacting the CTC resin with a N-protected amino acid, such as FMOC-Leucine in the presence of a non-nucleophilic base such as diisopropylethyl amine. DCM was typically the solvent of choice. This loading lead to an initial amino acid linked to the resin through the 2' chlorotrityl group. The moisture sensitive and expensive to produce chloride was only incorporated in the CTC to activate the resin to react with the amino acid. It has previously been taught that chlorotrityl resins must be converted to the chloride form for loading (US 5198531) None of the art teaches or fairly suggests the direct loading of a polymer bound substituted or unsubstituted trityl alcohol.

### SUMMARY OF THE INVENTION

It is an object of the present invention to provide a process for making a biologically active substance or therapeutic agent free of a chlorotrityl chloride linker-resin. The process includes reacting an activated N-protected amino acid or activated amino acid derivative with a substituted or unsubstituted trityl alcohol resin to obtain a resin-CT-AA product; deprotecting; and, then reacting the resin-CT-AA product with other building blocks of the biologically active substance or therapeutic agent to obtain the biologically active substance or therapeutic.

In one embodiment, a product created by this process is also provided herein. In yet a further variant, a substrate for creating a therapeutic or other biologically active substance is provided.

These and other objects of the present invention will become apparent from the detailed description of the invention, and from other portions of the specification.

### DETAILED DESCRIPTION OF THE INVENTION

The present invention provides a method for producing solid supported amino acid resins that overcome the problems in the art and meets the demand for a robust process to produce large scale quantities. Further, the method of the present invention results in substantially decreased cycle times, decreased raw material usage and reduced handling issues compared to methods using CTC loaded resins. Many of the advantages of the present invention and test results obtained were unexpected.

In one variant, the invention provides a process for making a biologically active substance or therapeutic agent free of a chlorotrityl chloride linker-resin or other halogen containing linker-resin. The process includes reacting an activated N-protected amino acid or activated amino acid derivative with a substituted or unsubstituted trityl alcohol resin to obtain a resin-CT-AA-PG ("protecting group") product. The resin-CT-AA-PG product is deprotected and reacted with other desired building blocks of the biologically active substance or therapeutic agent to obtain the biologically active substance or therapeutic.

In one embodiment, the N-protected activated amino acid is selected from the group consisting of an amino acid chloride, amino acid fluoride, amino acid bromide, an amino acid mixed anhydride, an amino acid activated ester, an amino acid containing a halogen, and preferably, an FMOC-amino acid chloride. The substituted or unsubstituted trityl alcohol resin used in the invention includes a chlorotrityl alcohol resin, methoxytrityl alcohol resin, dimethoxytrityl alcohol resin ethoxytrityl alcohol resin, and/or dimethoxytrityl alcohol resin. By way of example, the chlorotrityl alcohol resin is a 2'chlorotrityl alcohol resin.

In a further aspect, it is appreciated that the process described herein can include executing process steps to make the process FDA compliant. Various FDA compliance obtaining steps are known to those skilled in the art.

In another variant, the invention provides a product created by the process described herein. It is appreciated that because of the advantages of the process described herein, there is a significant and unexpected reduction in amino acid or amino acid derivative usage when creating a therapeutic agent or biologically active substance described herein. Moreover, more agent or substance can be created in a shorter time period since cycle times are reduced using the process described herein.

In yet another aspect, the invention includes a process for making a substrate upon which a therapeutic agent or other biologically active substance can be created. The process includes reacting an activated amino acid or derivative thereof with a substituted or unsubstituted trityl alcohol resin to obtain a resin-CT-AA product. The resin-CT-AA product is then used as a substrate for the addition of other desired components, including other amino acids, or other components described herein.

In yet another aspect, the invention provides a method to form a resin-CT-AA product. This resin is moisture stable and as it requires less steps to produce is less expensive. The process also generates less waste as compared to a CTC resin process. A method to produce the loaded resin directly from the intermediate 2'chlorotrityl alcohol resin is described. Typically, to join an alcohol to an amino acid one would use a coupling agent such as DIC, DCC, HBTU, TATU, PyBOP or other coupling agent. These activators are expensive, can be hazardous and are bulky slowing or stopping the reaction with the hindered trityl alcohol group. By using the FMOC-amino acid chloride in the presence of a non-nucleophilic base, it is possible to join the amino acid directly to the alcohol resin. The amino acid chloride is commercially available from Advanced ChemTech (Louisville, KY) or can be easily made by conventional techniques.

Solid supported 2'trityl alcohol can be commercially obtained from Aldrich Chemical, or it can be synthesized by methods known to those skilled in the art. See for example Orosz, Tetrahedron Letters, (39 ) 1998 at pg 3241-3241 wherein a cross-linked polystyrene bead can be converted to a polymer supported benzophenone with benzoyl chloride and a Lewis acid catalyst. The resultant benzophenone functionality is then transformed to the trityl alcohol functionality with phenyl lithium. The solid supported trityl alcohol is then ready to be converted via the process of the present invention to the amino acid loaded resin.

The solid supported trityl alcohol can be substituted or unsubstituted. The substituents include, but are not limited to, halogens, including but not limited to chloro, bromo and, fluro; substituted or unsubstituted alkoxy groups including but not limited to ethoxy, methoxy, propyloxy, methyleneoxy, ethyleneoxy, ethylene glycol, and propanediol; substituted or unsubstituted alkyl poly ether groups, including ,but not limited to, diethyleglycol, dipropanediol, triethyleneglycol, and tripropyleneglycol; substituted or unsubstituted lower alkyl groups having 1-6 carbon atoms including but not limited to methyl, ethyl, n-propyl, i-propyl, and trifluoromethyl; substituted or unsubstituted aryl groups including, but not limited to, phenyl, benzyl, tolyl, methoxyphenyl, and chlorophenyl, substituted or unsubstituted heteroaryls , and substituted or unsubstituted cycloalkanes.

Non-nucleophilic base compounds useful in the practice of the present invention include, but are not limited to Diisopropylethylamine (DIEA), triethylamine, pyridine, 2,4,6-collidine, DMAP, phenyl dimethyl amine other substituted amines and mixtures thereof. More preferred non-nucleophilic base compounds are Diisopropylethylamine (DIEA), triethylamine, and pyridine and mixtures thereof. The most preferred non-nucleophilic base compounds is triethylamine.

The preferred range of amide containing catalyst compound is 0.5 to 10.00 eq, the more preferred range is 0.7 to 5 eq , and the most preferred range is 1 to 3eq.

In one aspect of the invention, an expensive resin functionalization step and related washing step is eliminated. This method also replaces the use of moisture sensitive resin with limited shelf life, with a moisture stable and less expensive resin which has a much longer shelf life. Hence, the methods described herein provide a loaded resin that exhibits a longer, superior shelf life over conventional resins. It is appreciated that these advantages of the present invention are unexpected.

Typically peptide resins are endcapped after loading. This can be done with methanol giving the substituted or unsubstitutedtrityl methylether. This is performed to keep new peptide chains from growing groups which were not esterified with the first amino acid and destroying the yield purity. Employing the less reactive CTOH resin of the present invention allows peptides to be build without endcaping with methanol. In one variant of the invention, one needs no endcap as the CTOH is rather hindered, not activated, and does not react with typical bulky activated (DCC, HBTU, PyBOP, etc) amino acids. This also makes the resin more hydrophillic and increases the peptide/amino acid solubility within the polymer gel. In another variant of the invention, where the residual CTOH is found to be reactive, it is optionally endcapped with acetyl chloride, acetic anhydride or other desired endcapping compound.

It is appreciated that the present invention greatly impacts recycling of the resin, and provides for a resin that has increased recyclablity. This advantage is even more greatly provided if combined with a new low void space copolymer as described in U.S. Provisional Patent Application Serial No. 60/404,045, by Bohling et al., filed on August 16, 2002, entitled "Low Void Space Resin" (Docket number DN# A01406), incorporated by reference as if fully set forth herein.

Typically to recycle this resin one cleaves the peptide from the resin with dilute acid in organic solvent. The resultant trityl carbocation is then quenched with base to give CTOH resin, which is then washed and treated with SOCl2 or AcCl to regenerate a moisture sensitive CTC. This process is roughly outlined in US6239220 B1, incorporated herein as if fully set forth, and discussed in Harre et al. (Reactive and functional Polymers 41 (1999) 111-114). In addition, to the benefit of skipping the chlorination reaction and generating a moisture insensitve product, the method also avoids the requirement to cleave the encapping methoxy groups, allowing more mild cleavage condition and higher recycle yields.

An exemplary "amino acid" that can be used in the present invention, and loaded on the resin as described herein, is a compound represented by NH.sub.2-- CHR--COOH, wherein R is H, an aliphatic group, a substituted aliphatic group, an aromatic group or a substituted aromatic group. A "naturally-occurring amino acid" is found in nature. Examples include alanine, valine, leucine, isoleucine, aspartic acid, glutamic acid, serine, threonine, glutamine, asparagine, arginine, lysine, ornithine, proline, hydroxyproline, phenylalanine, tyrosine, tryptophan, cysteine, methionine and histidine. R is the side-chain of the amino acid. Examples of naturally occurring amino acid side-chains include methyl (alanine), isopropyl (valine), sec-butyl (isoleucine), --CH.sub.2CH(--CH).sub.2 (leucine), benzyl (phenylalanine), p-hydroxybenzyl (tyrosine), --CH.sub.20H (serine), CHOHCH.sub.3 (threonine), --CH.sub.2-3-indoyl (tryptophan), --CH.sub.2COOH (aspartic acid), CH.sub.2CH.sub.2COOH (glutamic acid), -- CH.sub.2C(O)NH.sub.2 (asparagine), --CH.sub.2CH.sub.2C(O)NH.sub.2 (glutamine), --CH.sub.SSH, (cysteine), --CH.sub.2CH.sub.2SCH.sub.3 (methionine), --(CH.sub.2).sub.4NH.sub.2 (lysine), --(CH.sub.2).sub.3NH.sub.2 (omithine), -[(CH).sub.2].sub.4NHC(.- dbd.NH)NH.sub.2 (arginine) and --CH.sub.2-3-imidazoyl (histidine). The side-chains of alanine, valine, leucine and isoleucine are aliphatic, i.e., contain only carbon and hydrogen, and are each referred to herein as "the aliphatic side chain of a naturally occurring amino acid."

The side chains of other naturally-occurring amino acids that can be used in the present invention include a heteroatom-containing functional group, e.g., an alcohol (serine, tyrosine, hydroxyproline and threonine), an amine (lysine, omithine, histidine and arginine), a thiol (cysteine) or a carboxylic acid (aspartic acid and glutamic acid). When the heteroatom-containing functional group is modified to include a protecting group, the side-chain is referred to as the "protected side-chain" of an amino acid.

The selection of a suitable protecting group depends upon the functional group being protected, the conditions to which the protecting group is being exposed and to other functional groups which may be present in the molecule. Suitable protecting groups for the functional groups discussed above are described in Greene and Wuts, "Protective Groups in Organic Synthesis", John Wiley & Sons (1991), the entire teachings of which are incorporated into this application by reference as if fully set forth herein. The skilled artisan can select, using no more than routine experimentation, suitable protecting groups for use in the disclosed synthesis, including protecting groups other than those described below, as well as conditions for applying and removing the protecting groups.

Examples of suitable alcohol protecting groups include benzyl, allyl, trimethylsilyl, tert-butyldimethylsilyl, acetate, and the like. Examples of suitable amino protecting groups include benzyloxycarbonyl, tert-butoxycarbonyl, tert-butyl, benzyl and fluorenylmethyloxycarbonyl (Fmoc). Tert-butoxycarbonyl is a preferred amine protecting group. Examples of suitable carboxylic acid protecting groups include tert-butyl, Fmoc, methyl, methoxylmethyl, trimethylsilyl, benzyloxyrnethyl, tert-butyldimethylsilyl and the like. Tert-butyl is a preferred carboxylic acid protecting group. Examples of suitable thiol protecting groups include S-benzyl, S-tert-butyl, S-acetyl, S-methoxymethyl, S-trityland the like.

Lysine, aspartate and threonine are examples of amino acid side-chains that are preferably protected in one variant of the invention. Aliphatic groups include straight chained, branched C.sub.1-C.sub.8, or cyclic C.sub.3-C.sub.8 hydrocarbons which are completely saturated or which contain one or more units of unsaturation. In one example, an aliphatic group is a C1-C4 alkyl group. Aromatic groups include carbocyclic aromatic groups such as phenyl, 1-naphthyl, 2-naphthyl, 1-anthracyl and 2-anthracyl, and heterocyclic aromatic groups such as N-imidazolyl, 2-imidazole, 2-thienyl, 3-thienyl, 2-furanyl, 3-furanyl, 2-pyridyl, 3-pyridyl, 4-pyridyl, 2-pyrimidy, 4-pyrimidyl, 2-pyranyl, 3-pyranyl, 3-pyrazolyl, 4-pyrazolyl, 5-pyrazolyl, 2-pyrazinyl, 2-thiazole, 4-thiazole, 5-thiazole, 2-oxazolyl, 4-oxazolyl and 5-oxazolyl.

Aromatic groups also include fused polycyclic aromatic ring systems in which a carbocyclic aromatic ring or heteroaryl ring is fused to one or more other heteroaryl rings. Examples include 2-benzothienyl, 3-benzothienyl, 2-benzofuranyl, 3-benzofuranyl, 2-indolyl, 3-indolyl, 2-quinolinyl, 3-quinolinyl, 2-benzothiazole, 2-benzooxazole, 2-benzimidazole, 2-quinolinyl, 3-quinolinyl, 1-isoquinolinyl, 3-quinolinyl, 1-isoindolyl, 3-isoindolyl, and acridintyl.

Suitable substituents for an aryl group and aliphatic group are those which are compatible with the disclosed reactions, i.e., do not significantly reduce the yield of the reactions and do not cause a significant amount of side reactions. Suitable substituents generally include aliphatic groups, substituted aliphatic groups, aryl groups, halogens, halogenated alkyl groups (e.g., trihalomethyl), nitro, nitrile, --CONHR, --CON(R).sub.2, --OR, --SR, --S(O)R, -- S(O).sub.2R, wherein each R is independently an aliphatic group, or an aryl group. Although certain functional groups may not be compatible with one or more of the disclosed reactions, these functional groups may be present in a protected form. The protecting group can then be removed to regenerate the original functional group. Skilled artisan will be able to select, using no more than routine experimentation, protecting groups which are compatible with the disclosed reactions.

A peptide mimetic, or component thereof, can also be used in the present invention, loaded onto a resin as described herein, or created by the process described herein. A peptide mimetic is a compound which has sufficient structural similarity to a peptide so that the desirable properties of the peptide are retained by the mimetic. For example, peptide mimetics used as protease inhibitors for treating HIV infection, are disclosed in Tung, et al., WO 94/05639, Vazquez, et al., WO 94/04491, Vazquez, et al., WO 94/10134 and Vaquez, et al., WO 94/04493. The entire relevant teachings of these publications are incorporated herein by reference. To be useful as a drug, a peptide mimetic should retain the biological activity of a peptide, but also have one or more properties which are improved compared with the peptide which is being mimicked. For example, some peptide mimetics are resistant to hydrolysis or to degradation in vivo. One strategy for preparing a peptide mimetic is to replace one or more amino acid residues in a peptide with a group which is structurally related to the amino acid residue(s) being replaced and which can form peptide bonds. The development of new amino acid derivatives which can be used to replace amino acid residues in peptides will advance the development of new peptide mimetic drugs.

Exemplary peptide mimetics are described in United States Patent Application Serial No. 20020188135 by Gabriel, Richard L. et al. filed on December 12, 2002 entitled, "Amino acid derivatives and methods of making the same." This patent application is incorporated by reference herein as if fully set forth. Also useful in the present invention are physiologically acceptable salts of these compounds. Salts of compounds containing an amine or other basic group can be obtained, for example, by reacting with a suitable organic or inorganic acid, such as hydrogen chloride, hydrogen bromide, acetic acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium and the like.

The present invention is also useful in the creation and manufacture of therapeutic agents and biologically active substances that have one or more peptides, peptide derivatives, or peptide mimetics as building blocks or constituents thereof. Compounds or fragments of a compound which are terminated with an ester functionality can also be created by the present invention. The therapeutic agent that can be manufactured or created using the invention can vary widely with the purpose for the composition. The agent(s) may be described as a single entity or a combination of entities. The delivery system is designed to be used with therapeutic agents having high water-solubility as well as with those having low water-solubility to produce a delivery system that has controlled release rates. The terms "therapeutic agent" and "biologically active substance" include without limitation, medicaments; vitamins; mineral supplements; substances used for the treatment, prevention, diagnosis, cure or mitigation of disease or illness; or substances which affect the structure or function of the body; or pro-drugs, which become biologically active or more active after they have been placed in a predetermined physiological environment.

Examples of useful therapeutic agents and biologically active substances include the following expanded therapeutic categories: anabolic agents, antacids, anti-asthmatic agents, anti-cholesterolemic and anti-lipid agents, anticoagulants, anti-convulsants, anti-diarrheals, anti-emetics, anti-infective agents, anti-inflammatory agents, anti-manic agents, anti-nauseants, anti-neoplastic agents, anti-obesity agents, anti-pyretic and analgesic agents, anti-spasmodic agents, anti-thrombotic agents, anti-uricemic agents, anti-anginal agents, antihistamines, anti-tussives, appetite suppressants, biologicals, cerebral dilators, coronary dilators, decongestants, diuretics, diagnostic agents, erythropoietic agents, expectorants, gastrointestinal sedatives, hyperglycemic agents, hypnotics, hypoglycemic agents, ion exchange resins, laxatives, mineral supplements, mucolytic agents, neuromuscular drugs, peripheral vasodilators, psychotropics, sedatives, stimulants, thyroid and anti-thyroid agents, uterine relaxants, vitamins, antigenic materials, and prodrugs.

Other examples of useful therapeutic agents and biologically active substances also include: (a) anti-neoplastics, antimetabolites, cytotoxic agents, immunomodulators; (b) anti-tussives; (c) antihistamines; (d) decongestants; (e) various alkaloids; (f) anti-arrhythmics; (g) antipyretics; (h) appetite suppressants; (i) expectorants; (j) antacids; (k) biologicals such as peptides, polypeptides, proteins and amino acids, hormones, interferons or cytokines and other bioactive peptidic compounds, such as HGH, tPA, calcitonin, ANF, EPO and insulin; (1) anti-infective agents such as anti-fungals, anti-virals, antiseptics and antibiotics; and (m) antigenic materials, partricularly those useful in vaccine applications.

The therapeutic agent or biologically active substance can, optionally, include a DNA or an RNA containing biologically active substance, a polysaccharide containing biologically active substance, growth factors, hormones, anti-angiogenesis factors, interferons or cytokines, and pro-drugs. The therapeutic agents created using the process described herein are used in amounts that are therapeutically effective. While the effective amount of a therapeutic agent will depend on the particular material being used, amounts of the therapeutic agent from about 1% to about 65% are useful. Lesser or greater amounts may be used to achieve efficacious levels of treatment for certain therapeutic agents.

### Example 1

### Loading of Surface-Functionalized Crosslinked Beads with Fmoc-L-Leucine Chloride

A 2-chlorotrityl alcohol resin produced according to U.S. Provisional Patent Application Serial No. 60/404,044, by Bohling et al., filed on August 16,2002, entitled "Resin for Solid Phase Synthesis" (Docket No. DN# A01407), incorporated herein as if fully set forth, is loaded with Fmoc-L-Leucine, treated with methanol to remove residual solvent and dried. The weight gain is used to quantify loading. The resin is assumed to have a capacity of 1.3 mmol/g. A portion of the resin is cleaved with 1% TFA/DCM, and the solution analyzed by HPLC to determine the cleaved yield (recovery) of amino acid.

Each sample of the resin (1.0000 +/- 0.05 g) is weighed into a 60 mL glass synthesizer vessel with a side port and a removable disk. The resin in the synthesizer is pre-swelled with dichloromethane (DCM). The DCM was drained and to each synthesizer is added a solution of Fmoc-L-Leu-Cl and diisopropylethylamine (DIEA) in 10 ml DCM. Slow nitrogen agitation is started. The quantities, in grams, of Fmoc-L-Leu-OH is 0.358, and of DIEA, in mL, is 0.177, per sample, respectively. Each mixture was allowed to react at ambient temperature for two hours, then the solution is drained. If necessary, any remaining trityl alcohol end groups can be capped by treatment for at least 30 minutes with DIEA (1 mL) and acetyl chloride (2 mL) in DCM (10 mL). Each sample of resin is washed with 5 x 10 mL portions of DCM and transferred to a tared 30 mL fritted glass funnel, then washed with another 2 x 10 mL portions of DCM. Each loaded resin is then de-swelled with 4 x 10mL portions of isopropanol (IPA) and partially dried by pulling air through the filter cake with vacuum, then drying the filter and resin overnight in a vacuum oven at 30°C. The filter and resin are then re-weighed and the difference in mass calculated. Mass of Leu = Final wt-(filter tare+1.000g resin).

### T-20 Example

This Example describes the preparation of a ten-peptide fragment of the peptide known as T-20, described in U.S. Pat. No. 6,015,881, Table 1, as Peptide No. 11, containing amino acids 17-26. U.S. Pat. No. 6,015,881 is incorporated herein by reference as if fully set forth. The kinetics of the reaction are followed by sampling resin periodically during the coupling and running a Kaiser test to determine the presence of any unreacted primary amine. The resin is compared with two competitive resins, one from Novabiochem, and the other from Polymer Labs.

A 2-chlorotrityl alcohol resin is loaded with Fmoc-L-Leucine Cl as in Example 1. A sample of the resin (1.0 g) is weighed into a 60 mL glass synthesizer vessel with a side port and a removable disk. DCM (10 mL) is charged to the vessel and agitated with nitrogen for 30 minutes, then drained. The leucine derivatized resin is then deprotected by charging 10 mL of a 25% solution of piperidine in N-methylpyrrolidone (NMP), agitating for 10 minutes, drained and repeating once. The deprotection residue is removed by washing with 7x10 mL volumes of NMP. The activated ester of next amino acid in sequence is prepared by dissolving 1.5 eq of amino acid (Fmoc-glu(t-Bu)-OH is the first added in this sequence, see table for charges and formula weights), 1.5 eq of 1-hydroxybenzotriazole (HOBT) (0.149 g) and 1.5 eq of DIEA (0.126 g) into 7.5 mL of NMP at room temperature. The solution is then chilled and 1.5 eq of O-benzotriazol-1-yl-N,N,N',N',-tetramethyluronium hexafluorophosphate (HBTU) (0.370 g) is added and stirred for 30 minutes. DCM (2.5 mL) is then charged to the solution and allowed to stand for 30 minutes. The activated amino acid solution is then charged to the drained resin and agitated with nitrogen. Samples are obtained and analyzed (Kaiser test) each 15 minutes and the results recorded. Upon completion of the reaction the resin is drained and washed with NMP (3x10 mL). This process is then repeated from the deprotection with piperidine for the rest of the amino acids in the sequence. (Glu(tBu), Lys(Boc), Asn(trt), Glu(tBu), Gln(trt), Glu(tBu), leu, leu, ). It is appreciated that the peptide synthesis cycle time using the method and components described in the present invention is greatly decreased as compared to other known techniques.

**Table 1:**

| Peptide Synthesis Efficiency Comparison | | | |
|---|---|---|---|
| Amino Acid | R+H | Polymer Nova Labs | Biochem |
| 1 | 45 | 60 | 60 |
| 2 | 30 | 60 | 60 |
| 3 | 60 | 60 | 60 |
| 4 | 30 | 45 | 45 |
| 5 | 60 | 60 | 60 |
| 6 | 30 | 45 | 45 |
| 7 | 30 | 45 | 30 |
| 8 | 30 | 45 | 30 |
| 9 | 30 | 45 | 45 |
| Total Cycle Time | 345 | 465 | 435 |
| | | | |

Results given in minutes to negative Kaiser Test

| Appendix 1 AA usage | | | |
|---|---|---|---|
| Monomer | fwt | g required | |
| FMOC Glu (t-Bu) | 425.48 | 0.415 | |
| FMOC Lys (Boc) | 468.55 | 0.457 | |
| FMOC Asn (trt) | 596.68 | 0.582 | |
| FMOC Glu (t-Bu) | 425.48 | 0.415 | |
| FMOC Gln (trt) | 610.71 | 0.595 | |
| FMOC Glu (t-Bu) | 425.48 | 0.415 | |
| FMOC Leu | 353.42 | 0.345 | |
| FMOC Leu | 353.42 | 0.345 | |
| FMOC Glu (t-Bu) | 425.48 | 0.415 | |
| HOBT | 153.15 | 0.149 | |
| DIEA | 129.25 | 0.126 | |
| HBTU | 379.25 | 0.370 | |
| LeuCT-resin (g)= | 1.00 | Total NMP | 1035.5mL |
| Loading Level (mmol/g)= | 0.65 | Total DCM | 72.5mL |
| Number of samples= | 1.00 | Total | 32mL |
| | | Piperdine | |
| Total resin(g)= | 1.00 | Total HOBT | 1.344g |
| Total mmol= | 0.65 | Total DIEA | 1.134G |
| Eq. of Monomer Charge= | 1.50 | Total HBTU | 3.328G |
| Coupling cycles per step= | 1.00 | | |
| Monomer usage/Cycle | 0.975 | | |
| (mmol)= | | | |
| AA Added | 9 | | |

It is appreciated that the methods described herein can be used for very low cost and efficient synthesis of peptides, in particular T-20, and T-20-like peptides. Such methods utilize solid and liquid phase synthesis procedures to synthesize and combine groups of specific peptide fragments to yield the peptide of interest. In other variant, individual peptide fragments which act as intermediates in the synthesis of the peptides of interest (e.g., T-20) are also created. In yet another aspect the present invention provides for the creation of groups of such peptide intermediate fragments which can be utilized together to produce full length T-20 and T-20-like peptides. One of ordinary skill in the art will appreciate that the cycle times for producing peptides, including but not limited to T-20 which include assembly of many smaller fragments, are in the aggregate also substantially reduced. Not only are cycle times be reduced but waste is greatly reduced, and efficiency is greatly increased.

In another aspect, the peptides or fragments of peptides created by the processes described herein are purified, and/or the individual peptide fragments which act as intermediates in the synthesis of the subject peptides are also purified.

It is further appreciated that the invention can also be used to create peptides and peptide fragments which exhibit an ability to inhibit fusion-associated events, and, importantly, also exhibit potent antiviral activity. These peptides and peptide fragments are described in U.S. Pat. Nos. 5,464,933; 5,656,480 and PCT Publication No. WO 96/19495, incorporated by reference herein as expressly set forth. The invention provides a method for creating these therapeutics in large scale quantities.

T-20 and T-20 fragments are made using solid and liquid phase synthesis procedures to synthesize and combine groups of specific peptide fragments to yield the peptide of interest. Generally, the methods of the invention include synthesizing specific side-chain protected peptide fragment intermediates of T-20 or a T-20-like peptide on a solid support created by the invention described herein, coupling the protected fragments in solution to form a protected T-20 or T-20-like peptide, followed by deprotection of the side chains to yield the final T-20 or T-20-like peptide. A preferred embodiment of the methods of the invention involves the synthesis of a T-20 peptide having an amino acid sequence as depicted in U.S. Patent No. 6,015,881 ("'881 Patent").

The present invention further relates to individual peptide fragments which act as intermediates in the synthesis of the peptides of interest (e.g., T-20). The peptide fragments of the invention include, but are not limited to, those having amino acid sequences as described in the '881 Patent.

It is appreciated that the present invention can also create one or more peptide fragments using conventional techniques using CTC-resins, and create one or more peptide fragments using the techniques described herein using the alcohol based resins. The resulting peptides can thereafter be combined to obtain the T-20 peptides or T-20 like peptides.

It will be understood that the methods, fragments and groups of fragments and techniques utilized for choosing the fragments and groups of fragments of the present invention may be used to synthesize T-20-like fragments in addition to T-20. The term "T-20-like" as used herein means any HIV or non-HIV peptide listed in U.S. Pat. Nos. 5,464,933; 5,656,480 or PCT Publication No. WO 96/19495, each of which is hereby incorporated by reference in its entirety.

In addition to T-20 and the T-20-like peptides described above, the methods, fragments and groups of fragments of the present invention may be used to synthesize peptides having modified amino and/or carboxyl terminal ends.

In a preferred embodiment, the methods of the invention are used to synthesize the peptide having a formula wherein X is an acetyl group and Z is an amide group. In a preferred method, T-20 and T-20-like peptides and intermediates can be purified using any non-silica based column packing (for maximization of loading capacity) including but not limited to zirconium-based packings, poly-styrene, poly-acrylic or other polymer based packings which are stable at high (greater than >7) pH ranges. For example, among the non-silica-laded column packing exhibiting a broad pH range that includes pH valves greater than that are sold by Tosohaus (Montgomeryville, Pa.). Columns packed with such material can be run in low, medium or high pressure chromatography

The present invention also provides for large scale efficient production of peptide fragment intermediates of T-20 and T-20-like peptides with specific amino acid sequences as listed in Table 1 above of the '881 Patent, and the groups of peptide fragment intermediates listed in Table 2 of the '881 Patent. Such peptide intermediates, especially in groups as listed in Table 2 of the '881 Patent are utilized to produce T-20 and T-20 like peptides.

Any one or more of the side-chains of the amino acid residues of peptide fragments may be protected with standard protecting groups such as t-butyl (t-Bu), trityl (trt) and t-butyloxycarbonyl (Boc). The t-Bu group is the preferred side-chain protecting group for amino acid residues Tyr(Y), Thr(T), Ser(S) and Asp(D); the trt group is the preferred side-chain protecting group for amino acid residues His(H), Gln(Q) and Asn(N); and the Boc group is the preferred side-chain protecting group for amino acid residues Lys(K) and Trp(W).

During the synthesis of fragments, the side-chain of the histidine residue is be protected, preferably with a trityl (trt) protecting group. If it is not protected, the acid used to cleave the peptide fragment from the resin may detrimentally react with the histidine residue, causing degradation of the peptide fragment.

The glutamine residues of the peptide fragments of the invention are protected with trityl (trt) groups. However, it is possible not to protect the glutamine residue at the carboxy-terminal end of certain fragments. All the asparagine residues of each peptide fragment of the invention can be protected. In addition, the tryptophan residue is protected with a Boc group.

Some of the individual peptide fragments are made using solid phase synthesis techniques described herein, while other peptides of the invention are optionally made using a combination of solid phase and solution phase synthesis techniques. The peptides of the invention may alternatively be synthesized such that one or more of the bonds which link the amino acid residues of the peptides are non-peptide bonds. These alternative non-peptide bonds may be formed by utilizing reactions well known to those in the art, and may include, but are not limited to imino, ester, hydrazide, semicarbazide, and azo bonds, to name but a few.

In yet another embodiment of the invention, T-20 and T-20 like peptides comprising the sequences described above may be synthesized with additional chemical groups present at their amino and/or carboxy termini, such that, for example, the stability, reactivity and/or solubility of the peptides is enhanced. For example, hydrophobic groups such as carbobenzoxyl, dansyl, acetyl or t-butyloxycarbonyl groups, may be added to the peptides' amino termini. Likewise, an acetyl group or a 9-fluorenylmethoxy-carbonyl group may be placed at the peptides' amino termini. Additionally, the hydrophobic group, t-butyloxycarbonyl, or an amido group may be added to the peptides' carboxy termini. Similarly, a para-nitrobenzyl ester group may be placed at the peptides' carboxy termini.

Further, T-20 and T-20-like peptides may be synthesized such that their steric configuration is altered. For example, the D-isomer of one or more of the amino acid residues of the peptide may be used, rather than the usual L-isomer.

Still further, at least one of the amino acid residues of the peptides of the invention may be substituted by one of the well known non-naturally occurring amino acid residues. Alterations such as these may serve to increase the stability, reactivity and/or solubility of the peptides of the invention.

Preferably, one or more the peptide fragments of the present invention are synthesized by solid phase peptide synthesis (SPPS) techniques described herein using standard FMOC protocols. See, e.g., Carpino et al., 1970, J. Am. Chem. Soc. 92(19):5748-5749; Carpino et al., 1972, J. Org. Chem. 37(22):3404-3409. In another variant of the invention, one or more fragments are made using the solid phase synthesis of the peptide fragments of the present invention is carried out on super acid sensitive solid supports which include, but are not limited to, 2-chlorotrityl chloride resin (see, e.g., Barlos et al., 1989, Tetrahedron Letters 30(30):3943-3946) and 4-hydroxymethyl-3-methoxyphenoxybutyric acid resin (see, e.g., Seiber, 1987, Tetrahedron Letters 28(49):6147-6150, and Richter et al., 1994, Tetrahedron Letters 35(27):4705-4706). Both the 2-chlorotrityl chloride and 4-hydroxymethyl-3-methoxyphenoxy butyric acid resins may be purchased from Calbiochem-Novabiochem Corp., San Diego, Calif.

General procedures for production and loading of resins using conventional techniques can be used in addition to, or in combination with, the novel techniques described herein. Some fragments can be made using resin loading performed, for example, via the following techniques: The resin, preferably a super acid sensitive resin such as 2-chlorotrityl resin, is charged to the reaction chamber. The resin is washed with a chlorinated solvent such as dichloromethane (DCM). The bed is drained and a solution of 1.5 equivalents of an amino acid and 2.7 equivalents of diisopropylethylamine (DIEA) in about 8-10 volumes of dichloroethane (DCE) is added. The N-terminus of the amino acid should be protected, preferably with Fmoc, and the side chain of the amino acid should be protected where necessary or appropriate. The mixture is agitated with nitrogen bubbling for 2 hours. After agitation, the bed is drained and washed with DCM. The active sites on the resin are endcapped with a 9:1 MeOH:DIEA solution for about 20-30 minutes. The bed is drained, washed 4 times. with DCM and dried with a nitrogen purge to give the loaded resin.. The fragment is then built following standard washing, deprotecting, coupling and cleaving protocols. Other fragments are made using the novel techniques described herein. The fragments made by the various techniques are then combined as described.

Fmoc is the preferred protecting group for the N-terminus of the amino acid. Depending on which amino acid is being loaded, its side chain may or may not be protected. For example, when Trp is loaded, its side chain should be protected with Boc. Similarly, the side-chain of Gln may be protected with trt. However, when Gln is being loaded in preparation for the synthesis of the 1-16 peptide fragment, its side chain should not be protected. It is not necessary to protect the side-chain of Leu.

The Fmoc-protected amino acids used in loading the resin and in peptide synthesis are available, with or without side-chain protecting groups as required, from Sean or Genzyme. Other exemplary peptides and fragments described in U.S. Patent No. 6,281,331 (incorporated by reference herein as if fully set forth) can be made using the novel techniques described herein, alone or in combination with other conventional techniques.

### Example 2

### Synthesis of Side Chain Protected Fragment 24-33 of Human Calcitonin (Boc-Cys(Trt)-Gly-Asn(Trt)-Leu-Ser(tBu)-Thr-(tBu)-Cys(Trt)-Met-Leu-Gly-OH).

A 2-chlorotrityl alcohol resin produced according to Patent DN# A01407, U.S. Provisional Patent Application Serial No. 60/404,044, filed 8/16/2002 by Bohling et. al, entitled "Resin for Solid Phase Synthesis" (incorported herein by reference as if fully set forth) is loaded with Fmoc-L-Glycine, treated with methanol to remove residual solvent and dried. The weight gain is used to quantify loading. The resin is assumed to have a capacity of 1.3 mmol/g. A portion of the resin is cleaved with 1% TFA/DCM, and the solution analyzed by HPLC to determine the cleaved yield (recovery) of amino acid.

### Initial Loading

Each sample of the resin (1.0000 +/- 0.05 g) is weighed into a 60 mL glass synthesizer vessel with a side port and a removable disk. The resin in the synthesizer is pre-swelled with dichloromethane (DCM). The DCM was drained and to each synthesizer is added a solution of Fmoc-L-Gly-Cl and diisopropylethylamine (DIEA) in 10 ml DCM. Slow nitrogen agitation is started. The quantities, in grams, of Fmoc-L-Gly-OH is 0.358, and of DIEA, in mL, is 0.177, per sample, respectively. Each mixture was allowed to react at ambient temperature for two hours, then the solution is drained. If necessary, any remaining trityl alcohol end groups can be capped by treatment for at least 30 minutes with DIEA (1 mL) and acetyl chloride (2 mL) in DCM (10 mL). Each sample of resin is washed with 5 x 10 mL portions of DCM and transferred to a tared 30 mL fritted glass funnel, then washed with another 2 x 10 mL portions of DCM. Each loaded resin is then de-swelled with 4 x 10mL portions of isopropanol (IPA) and partially dried by pulling air through the filter cake with vacuum, then drying the filter and resin overnight in a vacuum oven at 30°C. The filter and resin are then re-weighed and the difference in mass calculated. Mass of Gly = Final wt-(filter tare+1.000g resin).

### Peptide Build

A 2-chlorotrityl alcohol resin is loaded with Fmoc-L-Gly-Cl as in example 1. A sample of the resin (1.0 g) is weighed into a 60 mL glass synthesizer vessel with a side port and a removable disk. DCM (10 mL) is charged to the vessel and agitated with nitrogen for 30 minutes, then drained. The glycine derivatized resin is then deprotected by charging 10 mL of a 25% solution of Piperidine in N-methylpyrrolidone (NMP), agitating for 10 minutes, drained and repeating once. The deprotection residue is removed by washing with 7x10 mL volumes of NMP. The activated ester of next amino acid in sequence is prepared by dissolving 1.5 eq of amino acid (Fmoc-Leu-OH is the first added in this sequence, see table for charges and formula weights), 1.5 eq of 1-hydroxybenzotriazole (HOBT) (0.149 g) and 1.5 eq of DIEA (0.126 g) into 7.5 mL of NMP at room temperature. The solution is then chilled and 1.5 eq of O-benzotriazol-1-yl-N,N,N',N',-tetramethyluronium hexafluorophosphate (HBTU) (0.370 g) is added and stirred for 30 minutes. DCM (2.5 mL) is then charged to the solution and allowed to stand for 30 minutes. The activated amino acid solution is then charged to the drained resin and agitated with nitrogen. Samples are obtained and analyzed (Kaiser test) each 15 minutes and the results recorded. Upon completion of the reaction the resin is drained and washed with NMP (3x10 mL). This process is then repeated from the deprotection with piperidine for the rest of the amino acids in the sequence. (Met, Cys(Trt), Thr(tBu), Ser(tBu)Leu, Asn(Trt), Gly, Cys(Trt)).

The peptide is then cleaved by adding 14 mL of 1%TFA in DCM and mixing for 5 minutes. The cleavage solution is then collected into a flask containing an equal amount of pyridine to the TFA. The cleavage is repeated and the 2 cleavage solutions combined. The solvent is exchanged for ethanol and the product precipitated by the addition of water. The solids are collected and washed with water then dried under vacuum at room temperature.

Using the methods described herein three (3) fragments of human calcitonin have been created. The three exemplary fragments have the following sequences have been created:
Fragment 1 HGln(Trt)-Thr(tBu)-Ala-Ile-Gly-Val-Gly-Ala-Pro-Gly-CTC Fragment 2 
Fmoc-Thr(tBu)-Tyr(tBu)-Thr(tBu)-Gln(tBu)-Asp(OtBu)-Phe-Asn(Trt)-Lys(Boc)-
Phe-His(Trt)-Thr(tBu)-Phe-Pro-OH
 Fragment 3

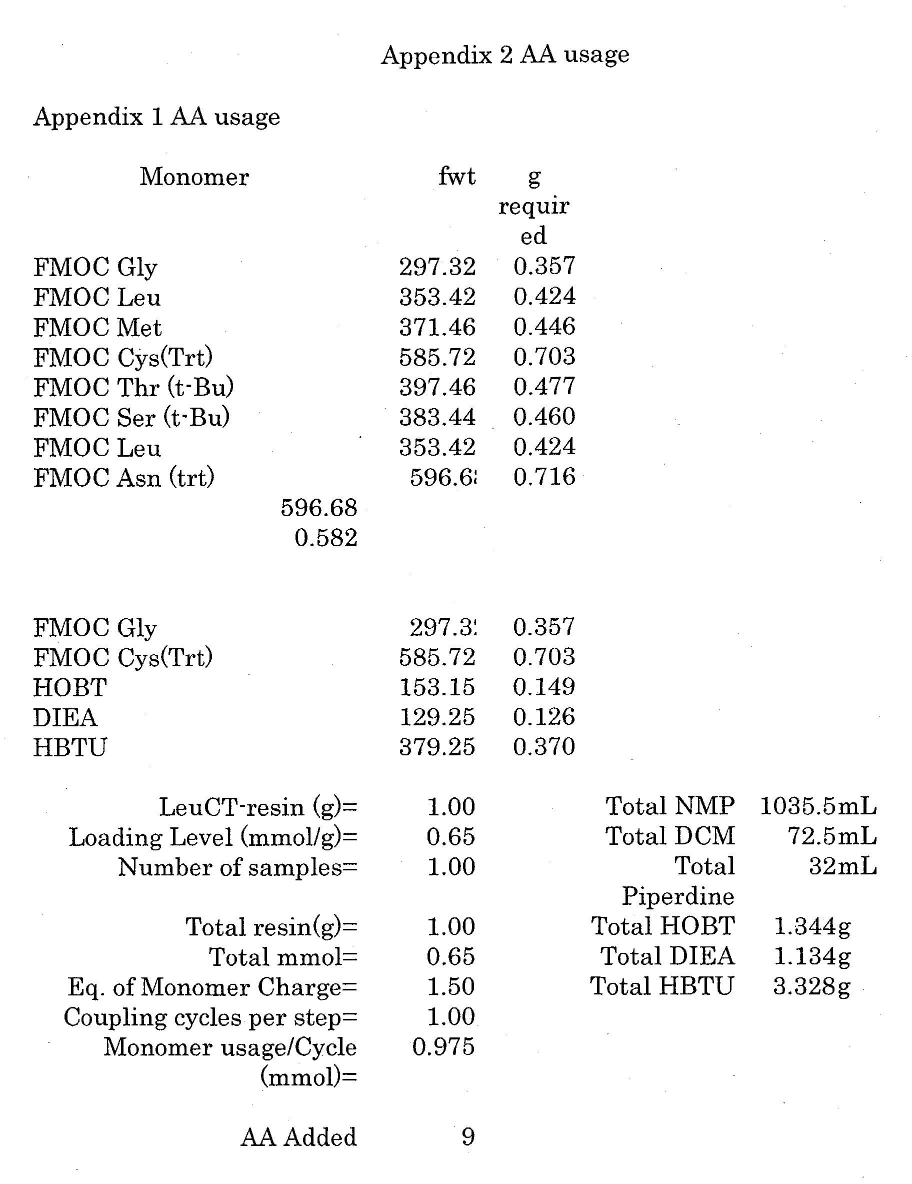

Boc-Cys(Trt)-Gly-Asn(Trt)-Leu-Ser(tBu)-Thr-(tBu)-Cys(Trt)-Met-Leu-Gly-OH

### Example 3

### Synthesis of T-1249

The processes and substrates described herein can also be used to construct the polypeptide described in U.S. Patent No. 6,469,136 ("'136 Patent"), incorporated herein by reference as if fully set forth. In particular, peptides referred to herein as T-1249 and T-1249-like peptides can be constructed using the novel methods described herein, alone or in combination with the conventional methods described herein. These methods utilize solid and liquid phase synthesis procedures to synthesize and combine groups of specific peptide fragments to yield the peptide of interest.

Novel methods for the synthesis of peptides, in particular peptides referred to herein as T-1249 and T-1249-like peptides, are described herein. These methods utilize solid and liquid phase synthesis procedures to synthesize and combine groups of specific peptide fragments to yield the peptide of interest. Generally, the methods include synthesizing specific side-chain protected peptide fragment intermediates of T-1249 or a T-1249-like peptide on a solid support, coupling the protected fragments in solution to form a protected T-1249 or T-1249-like peptide, followed by deprotection of the side chains to yield the final T-1249 or T-1249-like peptide. A preferred embodiment of the methods of the invention involves the synthesis of a T-1249 peptide having an amino acid sequence as depicted in the '136 Patent.

The present invention further provides a low cost, highly efficient method to construct individual peptide fragments which act as intermediates in the synthesis of the peptides of interest (e.g., T-1249). The peptide fragments of the invention include, but are not limited to, those having amino acid sequences as depicted in Table 1 of the '136 Patent.

Combinations of solid phase liquid phase synthetic reactions as described herein allow high purity T-1249 and T-1249-like peptides to be manufactured for on a large scale with higher throughput and higher yield than those described in the art. T-1249 and T-1249-like peptides may be synthesized on a scale of one or more kilograms.

### Creation of Full-length Peptides

The present invention is used to synthesize the peptide known as T-1249. T-1249 is a 39 amino acid residue polypeptide whose sequence is derived from HIV-1, HIV-2 and SIV gp41 viral polypeptide sequences. It will be understood that the methods, fragments and groups of fragments and techniques utilized for choosing the fragments and groups of fragments of the present invention may be used to synthesize T-1249-like fragments in addition to T-1249. The term "T-1249-like" as used herein means any HIV or non-HIV peptide listed in International Application No. PCT/US99/11219, filed May 20, 1999, International Publication No. WO 99/59615 published Nov. 25, 1999, which is hereby incorporated by reference in its entirety.

In addition to T-1249 and the T-1249-like peptides described above, the methods, fragments and groups of fragments of the present invention may be used to synthesize peptides having modified amino and/or carboxyl terminal ends. or other polymer based packings which are stable at high and low pH ranges.

### Peptide Intermediates

One or more peptide fragment intermediates of T-1249 and T-1249-like peptides with specific amino acid sequences as listed in Table 1 of the '136 Patent, and one or more groups of peptide fragment intermediates listed in Table 2 of the '136 Patent are also constructed using the novel processes described herein, alone or in combination with other art processes.

### Peptide Synthesis

Individual peptide fragments are preferably made using solid phase synthesis techniques, while other peptides of the invention are optionally made using a combination of solid phase and solution phase synthesis techniques. The syntheses culminate in the production of T-1249 or T-1249-like peptides.

The peptides of the invention may alternatively be synthesized such that one or more of the bonds which link the amino acid residues of the peptides are non-peptide bonds. These alternative non-peptide bonds may be formed by utilizing reactions well known to those in the art, and may include, but are not limited to imino, ester, hydrazide, semicarbazide, and azo bonds, to name but a few. Further, T-1249 and T-1249-like peptides may be synthesized such that their steric configuration is altered. For example, the D-isomer of one or more of the amino acid residues of the peptide may be used, rather than the usual L-isomer.

Still further, at least one of the amino acid residues of the peptides of the invention may be substituted by one of the well known non-naturally occurring amino acid residues. Alterations such as these may serve to increase the stability, reactivity and/or solubility of the peptides of the invention. Any of the T-1249 or T-1249-like peptides may be synthesized to additionally have a macromolecular carrier group covalently attached to its amino and/or carboxy termini. Such macromolecular carrier groups may include, for example, lipid-fatty acid conjugates, polyethylene glycol, carbohydrates or additional peptides.

Amino acid loaded resins are prepared using the novel techniques described herein. After agitation, the bed is drained and washed with DCM.. The bed is drained, washed four times with DCM and dried with a nitrogen purge to give the loaded resin.

Fmoc is the preferred protecting group for the N-terminus of the amino acid. Depending on which amino acid is being loaded, its side chain may or may not be protected. For example, when tryptophan (Trp) is loaded, its side chain should be protected with Boc. However, it is not necessary to protect the side-chain of leucine (Leu). Preferably, glutamic acid (Glu), aspartic acid (Asp), threonine (Thr) and serine (Ser) are protected as t-butyl ethers or t-butyl esters, and tryptophan (Trp) and lysine (Lys) are protected as t-butoxycarbonyl carbamates (Boc). The amide side-chain of asparagine (Asn) and glutamine (Gln) may or may not be protected with trityl groups.

Meanwhile, the subsequent amino acid in the sequence to be added to the resin is activated for reaction at its carboxy terminus. The amine terminus of each amino acid should be protected with Fmoc. Depending on which amino acid is being added, its side chain may or may not be protected. Preferably, the side-chains of tyr(Y), Thr(T), Ser(S), Glu(E) and Asp(P) are protected with t-Bu, the side-chains of Gln(Q) and Asn(N) are protected with trt, and the side-chains of Lys(K) and Trp(w) are protected with Boc. It is not necessary for the side-chains of Leu or Ile to be protected.

The amino acid can be activated as follows. The Fmoc-protected amino acid (1.5 eq), 1-hydroxybenzotriazole hydrate (HOBT) (1.5 eq), and diisopropylethylamine (DIEA) (1.5 eq) are dissolved in a polar, aprotic solvent such as N-methyl pyrrolidinone (NMP), dimethyl formamide (DMF) or dimethyl acetamide (DMAC) (about 7.5 vol.) at room temperature. The solution is chilled to 0-5 degrees C and then O-benzotriazol-1-yl-N,N,N',N'-tetramethyluronium hexafluorophosphate (HBTU) or O-benzotriazol-1-yl-tetramethyltetrafluoroborate (TBTU)(1.5 eq) is added followed by stirring for 5-15 minutes to dissolve. It is important that activation is carried out at 0-5 degrees C to minimize racemization of the amino acid. The HBTU is the last reagent added to the cold solution since activation and racemization cannot take place in its absence.

The solution of activated amino acid is charged to the drained resin, washing in with DCM (approximately 2.5 vol). Note that activation of the amino acid is carried out in NMP due to the insolubility of HBTU in DCM. However, DCM is added to the reaction at this point to maintain adequate swelling of the resin beads. The reaction is agitated with N.sub.2 bubbling for about 1 hour at 20-30 degrees. C.

If the resin is to be stored overnight between coupling cycles, the resin bed may be drained and covered with NMP under a nitrogen blanket. Alternatively, the bed may be drained, stored under a nitrogen blanket, then conditioned with a DCM wash prior to proceeding with the next coupling cycle. If the completed fragment is to be stored overnight prior to cleavage, the resin bed should be washed free of NMP with IPA because significant Fmoc deprotection can occur in NMP.

After the coupling is judged complete, the resin is drained and washed with 3 aliquots (approximately 10 vol.) of NMP. The cycle is repeated for subsequent mers (i.e., amino acids) of the peptide fragment. Following the final coupling reaction, the resin is washed with 4 aliquots (about 10 vol.) of NMP, then with 2 aliquots (approximately 10 vol.) of DCM and 2 IPA. The resin-bound peptide may be dried with a nitrogen purge or in an oven.

Peptides synthesized via solid phase synthesis techniques can be cleaved and isolated according to, for example, the following non-limiting techniques: The peptide may be cleaved from the resin using techniques well known to those skilled in the art. For example, solutions of 1% or 2% trifluoroacetic acid (TFA) in DCM or a combination of a 1% and a 2% solution of TFA in DCM may be used to cleave the peptide. Acetic acid (HOAC), hydrochloric acid (HCl) or formic acid may also be used to cleave the peptide. The specific cleavage reagent, solvents and time required for cleavage will depend on the particular peptide being cleaved. After cleavage the cleavage fractions are subjected to standard work-up procedures to isolate the peptide. Typically, the combined cleavage fractions are concentrated under vacuum, followed by reconstitution with polar aprotic or polar aprotic solvents such as ethanol (EtOH), methanol (MeOH), isopropyl alcohol (IPA), acetone, acetonitrile (ACN), dimethyl formamide (DMF), NMD, DMAC, DCM, etc., followed by precipitation or crystallization with antisolvent such as water or hexanes, and collection by vacuum filtration. Alternatively, the product may be triturated with organic solvents or water after isolation of the peptide.

For synthesis of full length T-1249 peptides, the peptide intermediates, can be coupled together to yield the T-1249 peptide. For example, the groups of peptide intermediates, above, can be coupled together to produce T-1249 full-length peptide using the one or more of the methods described herein.

In certain embodiments, a three fragment approach for synthesis of T-1249 can be followed. A "three fragment approach" synthesis refers to a T-1249 synthesis scheme which begins with three T-1249 intermediate peptide fragments that are synthesized and coupled using solid and liquid phase synthesis techniques into a full-length T-1249 peptide.

### Method for Solid Phase Peptide Synthesis (SPPS); General Procedure

A SPPS chamber is charged FmocLeu-resin (1 eq). The resin is conditioned in 5% piperidine DCM (7.5 vol) with a nitrogen purge for 15-30 minutes. The solvent is drained and the resin is treated with 2.times.20% piperidine in NMP (5 volumes) for 30 minutes to remove the Fmoc protecting group. After the second 20% piperidine/NMP treatment, the resin is washed with 5-7.times.NMP (5 vol) to a negative choranil test.

Meanwhile, the subsequent amino acid (1.5 eq), HOBT (1.5 eq) and DIEA (1.5 eq) are combined in 3:1 NMP/DCM (10 vol), allowed to fully dissolve at room temperature and cooled to 0 degrees C. HBTU is added, the solution is stirred for 10-15 minutes to dissolve the solid then added to the resin. The suspension is agitated with stirring under a nitrogen atmosphere for 1-3 hours. Coupling completion is monitored with a qualitative ninhydrin test. If the reaction is incomplete after 3 h (positive ninhydrin test persists) the reactor should be drained and a recoupling should be performed with a fresh solution of activated amino acid (0.5 eq). Normally after 30 min-1 h of recoupling a negative ninhydrin test is obtained. This cycle is repeated for the remaining amino acids in the fragment. As the fragment builds, the solvent volumes used in the washes may need to be increased from 5 volumes. Following the final coupling, the resin is washed with 3.times.5-8 volumes of NMP then 2.times.10 volumes of DCM and dried to constant weight in a vacuum oven at 40 degrees C.

### Preferred Methods for Cleavage of the Peptide from Resin

The methods below describe the cleavage of peptide AcAA1-12OH from the resin. However, the same methods may be used for cleavage of other peptide fragments of the present invention.

### Method A: Use of HOAc

The resin (1 g, 0.370 mmol) was treated with mixture of AcOH/MeOH/DCM (5:1:4, 20 vol, 20 mL) with nitrogen agitation for 1.5 h and the solution was transferred to a round bottom flask, stirred, and treated with water (20 vol). The resulting white slurry was concentrated (rotavap, 40 degrees. C bath) to remove DCM and the product collected by filtration. Drying to a constant weight affords 0.69 g (74%) of AcAA1-12OH in 87A % purity. A second treatment of the resin as above provided an additional 0.08 g (8.5%) of AcAA1-12OH of less pure material (83 Area %) suggesting a desired reaction time of slightly > 1.5 hr.

### Method B: Use of TFA

The resin (1 wt., 20 g) is washed with 5-6.times.1.7 volumes of 1% TFA in DCM, 3-5 minutes each. The 1% TFA/DCM washes are collected in a flask containing pyridine (1:1 volume ratio with the TFA in the wash). The product containing washes are combined (600 mL, 30 vol) and the DCM is removed by distillation to a minimum pot volume (.about.1/3 the original volume). The vacuum is adjusted to maintain a pot temperature of 15-25 degrees C. Ethanol (6.5 vol) is added and the distillation is continued until the DCM is removed (as determined by an increase in the temperature of the distillate). Again the vacuum is adjusted to maintain a pot temperature of 15-20 degrees C. The final pot volume should be .about.8-9 volumes. The solution is cooled to 5-10 degrees C and water (6.5 vol) is added over 30 minutes to precipitate the AcAA1-12OH. The solid is collected by vacuum filtration and washes with water (2-3 vol). The slurry is stirred at 0-5 degrees. C for 30 minutes, the solids are collected by vacuum filtration and dried to constant weight to give 16.80 g of AcAA1-12OH in 90% yield and 84 Area % (A %) purity.

### SPPS of FmocAA27-38OH and Cleavage from the Resin

SPPS of FmocAA27-38OH was carried out as described above starting with 10 g of FmocTrp(Boc)OR loaded at 0.75 mmol/g. Cleavage method B was used (169/120/1, 78% yield, 87.9A %).

HPLC Conditions: Vydac C8, cat. No. 208TP54, 5 u, 300 A, 0.9 mL/min., 280 nm. A: 0.1% TFA/water, B: A mixture of 80% I-PrOH/20% Acetonitrile and 0.1% TFA. 60-80% B/30 min. Typical sample preparation: Dissolve 1 mg in 0.10 mL NMP, dilute with 1 mL Acetonitrile. Inject 20 .mu.L into a 20 .mu.L loop.

### Recycling of CTOH resin

This invention is especially useful for recycling of substituted trityl resins. Typically to recycle these resins one first cleaves the product from the resin with weak acid forming the unbound peptide and a chlorotrityl cation. The cation is then treated with a basic solution such as sodium hydroxide in methanol (See, e.g. US Patent No. 6,239,220 incorporated herein by reference as if fully set forth), washed and dried to regenerate the substituted trityl alcohol. The traditional method is to then activate the substituted trityl alcohol resin by converting it to a substituted trityl chloride resin. This step is problematic and presents a number of challenges which can be avoided if one activates the protected amino acid instead. The activation of the resin requires large equipment volume per resin volume because the resin typically swells 5-8cc/g. There are often material construction issues when washing the resin, e.g. the thionyl chloride often used for the conversion may damage a standard stainless steel filter requiring the use of a more exotic material to prevent corrosion. Glass lined reaction kettles are common. Chloride resistant filters are uncommon and expensive. The washing of the resin after the OH to Cl conversion also requires a great deal of solvent which must then be recycled or disposed of. Finally, the substituted trityl chloride resin product is then moisture sensitive leading to handling and drying issues. The present invention which utilizes the activation of the amino acid is much less of a challenge since a liquid product rather than a solid is used. The use of a filter is eliminated and the massive amount of wash solvent is eliminated or reduced. This is also more volumetrically efficient as the amino acid does not swell like the resin. Further, this invention does not require methoxy endcapping so recycling cleavage conditions are more mild. Also, the hydroxy groups make the resin more hydrophilic and improve transport of reagents and product through the gel phase.

While only a few, preferred embodiments of the invention have been described hereinabove, those of ordinary skill in the art will recognize that the embodiment may be modified and altered without departing from the central spirit and scope of the invention. Thus, the preferred embodiment described hereinabove is to be considered in all respects as illustrative and not restrictive, the scope of the invention being indicated by the appended claims, rather than by the foregoing description, and all changes which come within the meaning and range of equivalency of the claims are intended to be embraced herein.

## Claims

1. A process for making a biologically active substance, a fragment of a biologically active substance, a therapeutic agent, or a fragment of a therapeutic agent, free of a chlorotrityl chloride linker-resin, comprising:
reacting an activated amino acid or activated amino acid derivative with a substituted or unsubstituted trityl alcohol resin to obtain a resin-CT-AA product; and,
reacting said resin-CT-AA product with other building blocks of said biologically active substance, said therapeutic agent, or said fragments, to obtain said biologically active substance, said therapeutic agent, said fragment of said biologically active substance, or said fragment of said therapeutic agent.

2. The process of claim 1 in which said activated amino acid is selected from the group consisting of a protected amino acid chloride, a protected amino acid fluoride, a protected amino acid bromide, and a protected amino acid mixed anhydride, a protected amino acid activated ester, and FMOC-amino acid chloride.

3. The process of claim 1 in which said substituted or unsubstituted trityl alcohol resin is selected from the group consisting of chlorotrityl alcohol resin, a substituted trityl alcohol resin with an alkoxy, a substituted trityl alcohol resin with a halogen, a substituted trityl alcohol with a substituted alkyl group, and a substituted trityl alcohol with one or more groups bound to the aromatic rings of the trityl group.

4. The process of claim 3 in which said chlorotrityl alcohol resin is a 2'chlorotrityl alcohol resin.

5. The process of claim 1 further comprising cleaving one or more of said fragments, said biologically active substance, or said therapeutic agent.

6. The process of claim 1 further comprising recycling said resin.

7. A product created by the process of claim 1.

8. The product of claim 7 in which said biologically active substance or fragment thereof is selected from the group consisting of a fragment of T-20, T-20, a fragment of a T-20 like peptide, and a T-20 like peptide a fragment of T-1249, T-1249, a fragment of a T-1249 like peptide, and a T-1249 like peptide.

9. A process for making a substrate used to create a biologically active substance or therapeutic, comprising:
reacting an activated amino acid or derivative thereof with a substituted or unsubstituted trityl alcohol resin to obtain a resin-CT-AA product.

10. The process of claim 9 further comprising using said resin-CT-AA product to create a biologically active substance precusor, therapeutic precursor, said biologically active substance, or said therapeutic.

11. The process of claim 9 further comprising recycling said resin for use in a subsequent creation step.

12. The process of claim 1 in which said substituted or unsubstituted trityl alcohol resin comprises a low void space resin.
